# EUROPEAN PATENT APPLICATION

(11) **EP 3 141 210 A1**
(43) Date of publication of application: **15.03.2017**
(21) Application number: 15185140.9
(22) Date of filing: 14.09.2015
(51) Int. Cl.: A61C 8/00, A61B 17/16

(54) **BONE REAMER USED IN THE INTERNAL SINUS LIFTING PROCEDURE OF DENTAL IMPLANTATION**

(71) Applicant: Zhang, Chunyuan, Guangdong Province (CN)
(72) Inventor: Zhang, Chunyuan, Guangdong Province (CN)
(74) Representative: Lang, Christian

(57) **Abstract**

A bone reamer used in the internal sinus lifting procedure of dental implantation is disclosed. A working end of the bone reamer includes a reamer body having a cutting edge, and a front end protrusion at the front end of the reamer body with a smooth, round and blunt surface. The diameter of the front end protrusion is smaller than that of the reamer body. During the internal sinus lifting procedure, a pilot drill is used to penetrate the bone lamella at the maxillary sinus floor, continued with the bone reamer of the present invention to enlarge a hole of implantation. As the front end surface of the bone reamer is smooth, round and blunt, the mucous membrane is blocked without being scratched and damaged. The front end protrusion also guides the bone reamer not to shift from a pathway grinded by the pilot drill, for the guiding purpose.

## Description

### BACKGROUND OF THE INVENTION

### a) Field of the Invention

The present invention relates to a dental apparatus, and more particularly to a bone reamer 1 which is used in the internal sinus lifting procedure of dental implantation.

### b) Description of the Prior Art

After losing a maxillary rear tooth, a patient may suffer from alveolar bone resorption, maxillary sinus pneumatization and severe insufficiency of bone mass in the rear tooth area, resulting in the difficulty in repairing and implantation in this area. In the maxillary sinus floor lifting procedure, an autogenous bone or a bone substitute is implanted into the space between the bone wall and the sinus mucous membrane at the maxillary sinus floor, in order to increase effectively the bone height from the top of alveolar ridge to the sinus floor, so that enough bone mass can be supplied for the implantation of an implant.

The maxillary sinus floor lifting procedure is normally divided into the external sinus lifting procedure and the internal sinus lifting procedure. In the external sinus lifting procedure, a window is opened on the side wall of the maxillary sinus, and the mucous membrane at the maxillary sinus floor is ablated and pushed upward and inward under direct viewing. Next, a bone grafting material is implanted (or not implanted, as the height lifted will be smaller) between the mucous membrane and the maxillary sinus floor, to increase the bone mass between the maxillary sinus floor and the top of alveolar ridge.

On the other hand, in the internal sinus lifting procedure, the conventional surgery is to determine the distance from the top of alveolar ridge to the maxillary sinus floor precisely through the curve reconstruction from tomography images or the maxillary bone CT (Computed Tomography) scan, followed by preparing a hole of implantation with a bone drill. Next, when the hole is drilled to about 1∼2mm to the maxillary sinus floor, a specialized hoisting device is used instead to fracture the bone lamella at the sinus floor and then the bone lamella, which is connected with the mucous membrane, is pushed into the sinus cavity. This method can penetrate the mucous membrane easily.

For the existing internal sinus lifting procedure, the present inventor has designed an internal sinus lifting procedure wherein the bone lamella at the sinus floor is grinded off by the bone drill with the patient's head raising up backward to keep the mucous membrane intact. This method is called the internal sinus lifting procedure with the bone lamella being grinded off by the bone drill. Compared to the conventional procedure, this method is easier, has a higher success rate and wider adaptation and is safer. Upon implementation, four or five bone drills are needed to enlarge the hole of implantation continuously for one implant. As each stage of drilling, from small to large, will need to penetrate the bone lamella at the sinus floor, and the tip of the conventional bone drill is sharp and able to cut, there still exists a certain risk that the mucous membrane is damaged or even penetrated if the operation is inappropriate. Accordingly, although in comparison to the conventional internal sinus lifting procedure, the internal sinus lifting procedure with the bone lamella being grinded off by the bone drill, as disclosed by the present inventor, can reduce the perforation rate of the mucous membrane significantly (the perforation rate of the conventional internal sinus lifting procedure can reach to 10∼56%; however, the perforation rate of the internal sinus lifting procedure with the bone lamella being grinded off by the bone drill, as disclosed by the present inventor, is less than 10%), as each stage of drilling will need to penetrate the maxillary sinus floor, the existing bone drill, especially the bone reamer, should be improved.

### SUMMARY OF THE INVENTION

The primary object of the present invention is to provide a bone reamer which is used in the internal sinus lifting procedure of dental implantation. The said bone reamer is able to protect the mucous membrane from being scratched in the surgery, reducing significantly the risk in damaging and penetrating the mucous membrane at the maxillary sinus floor, in order to solve the technical issues in the prior art.

To solve the abovementioned technical issues, the technical means of the present invention is to provide the bone reamer that is used in the internal sinus lifting procedure of dental implantation. A working end of the said bone reamer includes a reamer body which is provided with a cutting edge for the cutting operation and a front end protrusion which is disposed at a front end of the reamer body and is provided with a smooth, round and blunt surface.

Furthermore, the diameter of the said front end protrusion is smaller than that of the reamer body.

Furthermore, the diameter of the said front end protrusion is 2∼5mm, and the diameter of the said reamer body is 3∼6mm.

Furthermore, the height of the said front end protrusion is smaller than or equal to 10mm.

Furthermore, the said front end protrusion is cylindrical, near cylindrical, semi-spherical, spherical or elliptical.

Furthermore, the said bone reamer is made of titanium alloy or stainless steel.

Furthermore, the said reamer body is in a spiral shape.

Furthermore, an outer edge of an end surface at which the front end of the said reamer body is connected with a root part of the said front end protrusion is provided with a cutting edge.

Upon implementation of the present invention, when performing the internal sinus lifting procedure, a pilot drill can be used first to penetrate the bone lamella at the maxillary sinus floor, followed by using the bone reamer disclosed by the present invention to continue enlarging the hole of implantation. As the front end surface of the bone reamer of the present invention is smooth, round and blunt, the mucous membrane can be blocked and prevented from scratching. In addition, the front end protrusion can guide the bone reamer not to shift from a pathway grinded by the pilot drill, for the guiding purpose.

To enable a further understanding of the said objectives and the technological methods of the invention herein, the brief description of the drawings below is followed by the detailed description of the preferred embodiments.

### BRIEF DESCRIPTION OF THE DRAWINGS

FIG. 1 shows a three-dimensional structural schematic view of a specific embodiment, according to the present invention.
FIG. 2 shows a top view of the specific embodiment of the present invention.

### DETAILED DESCRIPTION OF THE PREFERRED EMBODIMENTS

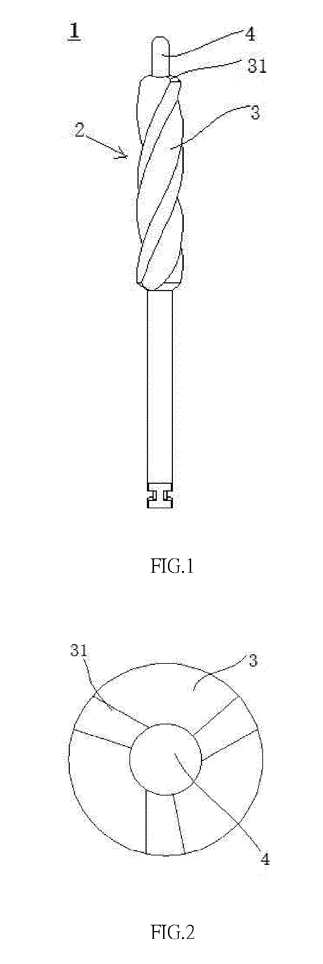

Referring to FIG. 1 and FIG. 2, the present invention discloses an embodiment of a bone reamer 1 used in the internal sinus lifting procedure of dental implantation. A working end 2 of the said bone reamer 1 includes a reamer body 3 which is provided with a cutting edge for the cutting operation, and a front end protrusion 4 which is disposed at the front end of the reamer body 3 and is provided with a smooth, round and blunt surface.

In performing the internal sinus lifting procedure, a pilot drill can be used to penetrate the bone lamella at the maxillary sinus floor, followed by using the bone reamer 1 of the present invention to continue enlarging the hole of implantation. As the surface of the front end of the bone reamer 1 of the present invention is smooth, round and blunt, the mucous membrane can be blocked and prevented from scratching. In addition, the front end protrusion 4 can guide the bone reamer 1 not to shift from the pathway grinded by the pilot drill, for the guiding purpose.

Specifically, after the pilot drill has penetrated the bone lamella at the maxillary sinus floor, as the diameter of the front end protrusion 4 is smaller than that of the reamer body 3, the reamer body 3 can be extended into the pathway grinded by the pilot drill without damaging the mucous membrane even that the mucous membrane is abutted. In addition, as the diameter of the reamer body 3 is larger than that of the front end protrusion 4 and the reamer body 3 is provided with the cutting edge for the cutting operation, the reamer body 3 can continue rotating and cutting to enlarge the hole of implantation. By the present embodiment, the flaw that the mucous membrane can be damaged easily when an existing bone reamer is enlarging the hole of implantation can be avoided. Besides that, in implementing the internal sinus lifting procedure with the bone lamella being grinded off by the bone drill, normally four or five bone reamers will be required to enlarge the hole of implantation continuously for a single implant, with that the diameter of the front end protrusion 4 of a next stage of bone reamer is smaller than that of the reamer body 3, but can be equal to or a little smaller than that of a previous stage of bone reamer. Thus, the front end protrusion 4 of the next stage of bone reamer can be extended into the hole of implantation enlarged by the previous stage of bone reamer, which also acts as the guiding purpose, enabling the next stage of bone reamer not to shift from the pathway grinded by the previous stage of bone reamer.

Preferably, the diameter of the said front end protrusion 4 is 2∼5mm, and the diameter of the said reamer body 3 is 3∼6mm. Naturally, these diameters may not be limited to the abovementioned ranges in other embodiments.

Preferably, the height of the front end protrusion 4 is smaller than or equal to 10mm.

In the present embodiment, the said front end protrusion 4 is in a shape of a cylinder. Naturally, the front end protrusion 4 can be near cylindrical, semi-spherical, spherical or elliptical in other embodiments, as long as that the front end protrusion 4 is provided with a smooth, round and blunt surface and can block the mucous membrane without damaging the mucous membrane easily.

Specifically, the said bone reamer 1 can be made of titanium alloy or stainless steel.

Specifically, the said reamer body 3 is in a spiral shape, and an outer edge at an end surface 31 at which the front end of the said reamer body 3 is connected with a root part of the said front end protrusion 4 is provided with a cutting edge. Therefore, when using the bone reamer 1, the front end protrusion 4, the surface of which is smooth, round and blunt, can hold the mucous membrane without scratching and damaging the mucous membrane easily. On the other hand, the cutting edge of the reamer body 3 can rotate and cut to enlarge the hole of implantation.

When implementing the bone reamer of the present invention, the risk in penetrating the mucous membrane at the maxillary sinus floor can be reduced significantly, because there is only a risk in penetrating the mucous membrane when the first pilot drill grinds through the bone lamella, and the following bone reamer can protect the mucous membrane from being scratched due to that the front end protrusion of the bone reamer is smooth, round and blunt. According to practicing, the perforation rate of the mucous membrane can reach to 10∼56% in the conventional internal sinus lifting procedure. However, based upon the internal sinus lifting procedure with the bone lamella being grinded off by the bone drill, as disclosed by the present inventor, the perforation rate of the mucous membrane can be decreased to less than 10% even using an existing beam reamer. Accordingly, with the bone reamer disclosed by the present invention, the perforation rate of the mucous membrane can be reduced significantly. Therefore, the present invention is provided with a very high value of being promoted to a clinical use.

It is of course to be understood that the embodiments described herein is merely illustrative of the principles of the invention and that a wide variety of modifications thereto may be effected by persons skilled in the art without departing from the spirit and scope of the invention as set forth in the following claims.

## Claims

1. A bone reamer used in the internal sinus lifting procedure of dental implantation, wherein a working end (2) of a bone reamer (1) includes a reamer body (3) having a cutting edge for a cutting operation, and a front end protrusion (4) disposed at front end of the reamer body (3) and having a smooth, round and blunt surface.

2. The bone reamer used in the internal sinus lifting procedure of dental implantation, according to claim 1, wherein diameter of the front end protrusion (4) is smaller than diameter of the reamer body (3).

3. The bone reamer used in the internal sinus lifting procedure of dental implantation, according to claim 2, wherein the diameter of the front end protrusion (4) is 2∼5mm, and the diameter of the reamer body (3) is 3∼6mm.

4. The bone reamer used in the internal sinus lifting procedure of dental implantation, according to claim 1, wherein a height of the front end protrusion (4) is smaller than or equal to 10mm.

5. The bone reamer used in the internal sinus lifting procedure of dental implantation, according to claim 1, wherein the front end protrusion (4) is cylindrical, near cylindrical, semi-spherical, spherical or elliptical.

6. The bone reamer used in the internal sinus lifting procedure of dental implantation, according to claim 1, wherein the bone reamer (1) is made of titanium alloy or stainless steel.

7. The bone reamer used in the internal sinus lifting procedure of dental implantation, according to claim 1, wherein the reamer body (3) is in a spiral shape.

8. The bone reamer used in the internal sinus lifting procedure of dental implantation, according to claim 7, wherein an outer edge at an end surface at which the front end of the reamer body is connected with a root part of the front end protrusion is provided with a cutting edge.
